(19) Europäisches Patentamt
European Patent Office
Office européen des brevets

(11) EP 1 079 797 B1

(12) FASCICULE DE BREVET EUROPEEN

(45) Date de publication et mention de la délivrance du brevet:
**14.12.2011 Bulletin 2011/50**

(21) Numéro de dépôt: **99923642.5**

(22) Date de dépôt: **28.05.1999**

(51) Int Cl.:
***A61K 8/34** (2006.01)* ***A61K 8/60** (2006.01)*
***A61K 8/63** (2006.01)* ***A61K 8/97** (2006.01)*
***A61Q 19/08** (2006.01)*

(86) Numéro de dépôt international:
**PCT/FR1999/001260**

(87) Numéro de publication internationale:
**WO 1999/062480 (09.12.1999 Gazette 1999/49)**

(54) **UTILISATION D'AU MOINS UNE SOYASAPONINE OU UN SOYASAPOGÉNOL EN ASSOCIATION AVEC UN ECDYSTÉROIDE, COMME AGENT COSMETIQUE DESTINE A AUGMENTER LA QUANTITE DE COLLAGENE IV DANS LA JONCTION DERMO-EPIDERMIQUE**

VERWENDUNG VON MINDESTENS EINEM SOJASAPONIN ODER SOJASAPOGENIN, IN KOMBINATION MIT EINEM ECDYSTEROID, ALS KOSMETISCHES MITTEL ZUR ERHÖHUNG DES TYP-IV-KOLLAGENGEHALTS IN DER GRENZSCHICHT VON DERMIS UND EPIDERMIS

USE OF AT LEAST A SOYSAPONIN OR SOYSAPOGENOL IN ASSOCIATION WITH AN ECDYSTEROID, AS COSMETIC AGENT FOR INCREASING THE AMOUNT OF COLLAGEN IV IN THE DERMAL-EPIDERMAL JUNCTION

(84) Etats contractants désignés:
**DE ES FR GB IT**

(30) Priorité: **29.05.1998 FR 9806821**

(43) Date de publication de la demande:
**07.03.2001 Bulletin 2001/10**

(73) Titulaire: **PARFUMS CHRISTIAN DIOR
75008 Paris (FR)**

(72) Inventeurs:
- **BONTE, Frédéric
F-45100 Orléans (FR)**
- **DUMAS, Marc,
61 Résidence le Windsor
F-45100 Orléans (FR)**
- **PERRIER, Pierre
F-45000 Orléans (FR)**

(74) Mandataire: **Portal, Gérard et al
Cabinet Beau de Loménie
158, rue de l'Université
75340 Paris Cedex 07 (FR)**

(56) Documents cités:
**EP-A- 0 826 367 EP-A- 0 826 367
WO-A-90/03778 WO-A-92/09262
WO-A-94/04132 WO-A-96/25143
WO-A-96/25143 WO-A-97/28814
WO-A-97/28814 WO-A-99/62481
US-A- 4 524 067**

- **GOMES DA SILVA, W: "The Brazil Nut (Bertholletia excelsa H.B.K.- Family: Lecythidaceae). Note II: Lipids: Study of the Chemical Composition." RIVISTA ITALIANA DELLE SOSTANZE GRASSE, vol. 74, no. 7, 1997, pages 311-314, XP000852534 Milano (IT)**
- **CHEMICAL ABSTRACTS, vol. 128, no. 4, 26 janvier 1998 (1998-01-26), Columbus, Ohio, US; abstract no.: 32365, EL-SHAMY, ALI M. ET AL: "Phytochemical study of Clerodendron inerme L. growing in Egypt" XP002124534 & ZAGAZIG J. PHARM. SCI., vol. 5, no. 2, 1996, pages 49-53,**
- **CHEMICAL ABSTRACTS, vol. 116, no. 5, 3 février 1992 (1992-02-03), Columbus, Ohio, US; abstract no.: 37930, R. ZAFAR: "Chemical examination of the leaves of Diospyros montana Roxb" XP002124547 & INDIAN DRUGS, vol. 28, no. 9, 1991, pages 28-29,**
- **A.Y LEUNG: "Encyclopedia of Common Natural Ingredients Used in Food, Drugs, and Cosmetics" 1996, WILEY-INTERSCIENCE PUBLICATION , NEW YORK (USA) , XP002099892 * page 284, colonne 1, alinéa 3 * * page 28, colonne 2, alinéa 3 * * page 285, colonne 1, ligne 2 ***

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 079 797 B1

- MASSIOT G ET AL: "Reinvestigation of the Sapogenins and Prosapogenins from Alfalfa (Medicago sativa )" JOURNAL OF AGRICULTURAL AND FOOD CHEMISTRY, AMERICAN CHEMICAL SOCIETY. WASHINGTON, US, vol. 36, no. 5, 1988, pages 902-909, XP009098573 ISSN: 0021-8561
- GOMES DA SILVA, W: "The Brazil Nut (Bertholletia excelsa H.B.K.- Family: Lecythidaceae). Note II: Lipids: Study of the Chemical Composition." RIVISTA ITALIANA DELLE SOSTANZE GRASSE, vol. 74, no. 7, 1997, pages 311-314, XP000852534 Milano (IT)
- CHEMICAL ABSTRACTS, vol. 128, no. 4, 26 janvier 1998 (1998-01-26) Columbus, Ohio, US; abstract no. 32365, EL-SHAMY, ALI M. ET AL: "Phytochemical study of Clerodendron inerme L. growing in Egypt" XP002124534 & ZAGAZIG J. PHARM. SCI. (1996), 5(2), 49-53,1996,
- CHEMICAL ABSTRACTS, vol. 116, no. 5, 3 février 1992 (1992-02-03) Columbus, Ohio, US; abstract no. 37930, R. ZAFAR: "Chemical examination of the leaves of Diospyros montana Roxb" XP002124547 & INDIAN DRUGS, vol. 28, no. 9, 1991, pages 28-29,
- A.Y LEUNG: "Encyclopedia of Common Natural Ingredients Used in Food, Drugs, and Cosmetics" 1996 , WILEY-INTERSCIENCE PUBLICATION , NEW YORK (USA) XP002099892 page 284, colonne 1, alinéa 3 page 28, colonne 2, alinéa 3 page 285, colonne 1, ligne 2

## Description

La présente invention concerne généralement l'utilisation de soyasaponines ou soyasapogénols en cosmétologie destinées au traitement de la peau en vue d'augmenter la quantité de collagène IV dans la jonction dermo-épidermique.

Elle concerne également de nouvelles compositions cosmétiques favorisant l'augmentation de la quantité de collagène IV dans la jonction dermo-épidermique ainsi qu'un procédé de traitement cosmétique utilisant une soyasaponine ou un soyasapogénol.

On sait que la jonction dermo-épidermique est une structure complexe qui assure la cohésion et les échanges entre le derme et l'épiderme, indispensables au bon fonctionnement de la peau.

On sait, par ailleurs, que le collagène de type IV, encore dénommé collagène IV est un constituant majeur de la jonction dermo-épidermique qui intervient notamment dans le maintien d'une interface fonctionnelle entre le derme et l'épiderme.

L'état physiologique optimum de la jonction dermo-épidermique dépend d'une quantité suffisante de collagène IV présente dans cette jonction dermo-épidermique.

Il est donc souhaitable, en vue de maintenir ou de restaurer l'état physiologique de la jonction dermo-épidermique, de disposer d'un moyen permettant d'y augmenter la quantité de collagène IV.

Il a été découvert, et ceci constitue le fondement de la présente invention, que les saponines ainsi que les sapogénols triterpéniques de type A ou de type B, en particulier cosmétiquement acceptables sont particulièrement appropriés en tant qu'agents permettant d'augmenter la quantité de collagène IV dans la jonction dermo-épidermique.

<u>DISCUSSION DE L'ART ANTERIEUR</u>

Il est connu par le document WO 96/25143 LVMH RECHERCHE l'utilisation d'extrait de Bertholletia excelsa dans le domaine cosmétique ou pharmaceutique. Ce document ne vise que l'utilisation de l'extrait global de Bertholletia excelsa.

Il est connu par le document GOMES DA SILVA et CORTESI (La Rivista Italiana delle Sostanze Grasse - 74 (7), 311-314 (1997)), que l'extrait de Bertholletia excelsa contient la β-amyrine et l'α-amyrine, qui ne sont pas des saponines ou sapogénols triterpéniques de type A ou de type B.

Il est aussi connu par CAS :volume 128, N°4, abstract N°32365a, décrit la composition d'extrait de Clerodendron inerme L. qui contient des saponines triterpéniques, dont les aglycones correspondantes sont l'acide oléanolique et la β-amyrine, ces deux molécules n'étant pas des sapogénines ou sapogénols triterpéniques de type A ou B. Aucune utilisation ou activité des produits n'est décrite.

Le document WO-A-97/28814 concerne l'utilisation d'extrait de plantes Uva Ursi et Lespedeza Capitata dans des compositions cosmétiques et pharmaceutiques comme agent anti-glycation des protéines afin de lutter contre le vieillissement cutané.

Les extraits des feuilles de la plante Uva Ursi contiennent des saponines ou sapogénols triterpéniques : acide ursolique, amyrine et lupéol, qui sont trois molécules de structures différentes des triterpènes de type A ou de type B, objet de la présente invention.

Le document CAS :vol 116, N°5, abstract N°37930, concerne la composition d'un extrait de la plante Diospyros montana Rox b, qui contient des saponines, et en particulier de l'acide ursolique, qui a une Structure différente des saponines ou sapogénols triterpéniques de type A ou B, objet de la présente invention.

Le document EP-A-0 826 367 LVMH RECHERCHE concerne une utilisation d'un extrait de Bertholletia dans le domaine cosmétique pour stimuler la synthèse de collagène VII et/ou pour favoriser l'incorporation de la vitamine C dans les cellules de la peau, en particulier dans les fibroblastes. Comme pour WO-A-96/25143, il s'agit de saponines différentes des saponines triterpéniques de type A ou B, objet de la présente invention.

Le document A.Y. LEUNG, Encyclopedia of Common Natural Ingredients used in foods, drugs and cosmetics, 1996, WILEY, Interscience publication, New York, pages 284-286, divulgue la composition d'un extrait de Centella asiatica et l'utilisation d'un tel extrait dans des compositions cosmétiques et dermatologiques, ces extraits de Centella asiatica contiennent des saponines triterpéniques, mais qui ne sont pas caractérisées comme étant de type A ou de type B, utilisées dans le cadre de la présente invention.

Le document WO 92/09 262 LVMH RECHERCHE décrit l'utilisation de saponines de medicago pour la préparation de composition cosmétique ou pharmaceutique, notamment dermatologique favorisant le renouvellement de l'épiderme, stimulant la repousse des cheveux ou retardant leur chute (voir titre et abrégé).

L'article de Georges MASSIOT dans J. Agric.Food Chem. 1988, 36, 902-909 décrit une étude sur les sapogénines et les Prosapogénines de Alfalfa (Medicago sativa).

Le document Arichi US 4,254,067 décrit de nouvelles saponines isolées de graines de soja avec leurs méthodes de séparation et leur utilisation comme agent antioxydant dans les produits alimentaires ou cosmétiques.

Le document LVMH RECHERCHE W0 94/04 132 décrit l'utilisation d'un ecdystéroïde en cosmétique ou en dermatologie pour donner à la peau un aspect plus lisse et plus doux, pour renforcer la fonction de barrière hydrique de la peau, ainsi que pour renforcer la cohésion des cellules de l'épiderme.

Le document LVMH RECHERCHE W0 90/03 778 décrit des phases lamellaires lipidiques hydratées ou liposomes à base d'ecdystéroïde et leur utilisation notamment dans une composition cosmétique.

Le document LVMH RECHERCHE WO 99/62 481 bénéficie de la même date de priorité du 29 mai 1998 que la présente demande et est cité pour mémoire.

Ainsi, selon un premier aspect, la présente demande vise à couvrir une utilisation non thérapeutique d'au moins une soyasaponine ou un soyasapogénol de nature triterpénique de type A ou de type B, cosmétiquement acceptable, en particulier une soyasaponine ou un soyasapogénol triterpénique de type A ou de type B extrait de plante, de préférence de soja ou d'une plante de type Medicago, ou d'un extrait végétal riche en de tels composés, en association avec au moins un ecdystéroïde ou avec au moins un dérivé acétylé d'ecdystéroïde, en particulier avec la beta-ecdysone ou ecdystérone, comme agent cosmétique destiné à augmenter la quantité de collagène IV dans la jonction dermo-épidermique, pour maintenir ou restaurer l'état physiologique de la jonction dermo-épidermique.

De très nombreuses soyasaponines et de très nombreux soyasapogénols de nature triterpénique, s'avèrent appropriés dans le cadre de l'utilisation conforme à la présente invention, dans le domaine cosmétique.

Avantageusement, ces composés, qui devront être cosmétiquement acceptables, dans le cadre d'une utilisation cosmétique, seront extraits de plantes. Ces composés pourraient être aussi obtenus par synthèse chimique, comme cela est bien compréhensible et connu de l'homme de l'art.

En particulier, on pourra utiliser des soyasaponines extraites de plantes, de préférence extraites de Glycine max (soja), de Phaseolus vulgaris, de Phaseolus aureus, de Phaseolus lunatus, de Vicia faba, de Lens culinaris, de Cicer arietum, de Vigna angularis, de Vigna mungo, de Oxytropis ochrocephala, d'Oxytropis glabra, de Pisum sativum, de Sophora flavescens, d'Asparalus membranaceus, de Crotalaria albida, d'Arachis hypogea, de Galega officinalis, de Wistaria brachybotrys, de Trifolium repens, ou extraites de plantes du type Medicago, en particulier Medicago alfalfa et Medicago sativa désigné souvent par le nom de "luzerne".

Le soja et les plantes de type Medicago telles que Medicago sativa sont des plantes particulièrement appropriés pour fournir des soyasaponines ou soyasapogénols utilisables dans le cadre de l'invention, puisqu'elles/ils sont de type triterpénique de type A ou de type B.

Avantageusement, ces soyasaponines ou soyasapogénols triterpéniques de type A ou de type B[, et] pourront être extrait(e)s en particulier d'une plante de type Medicago telle que Medicago alfalfa et Medicago sativa.

Ces soyasaponines ou soyasapogénols triterpéniques de type A ou de type B sont bien connus dans la littérature (voir d'une part l'article de Georges Massiot, Catherine Lavaud, Dominique Guillaume et Louisette Le Men-Olivier dans J. Agric. Food Chem. (1988), 36, pages 902-909, et d'autre part, l'ouvrage dénommé "Saponins" de Hostettman K. et Marston A. (1995), publié par Cambridge University Press, en particulier Appendix 2, références 446 à 460, 517 à 519. Leur formule chimique est la suivante :

- soyasapogénol de type A

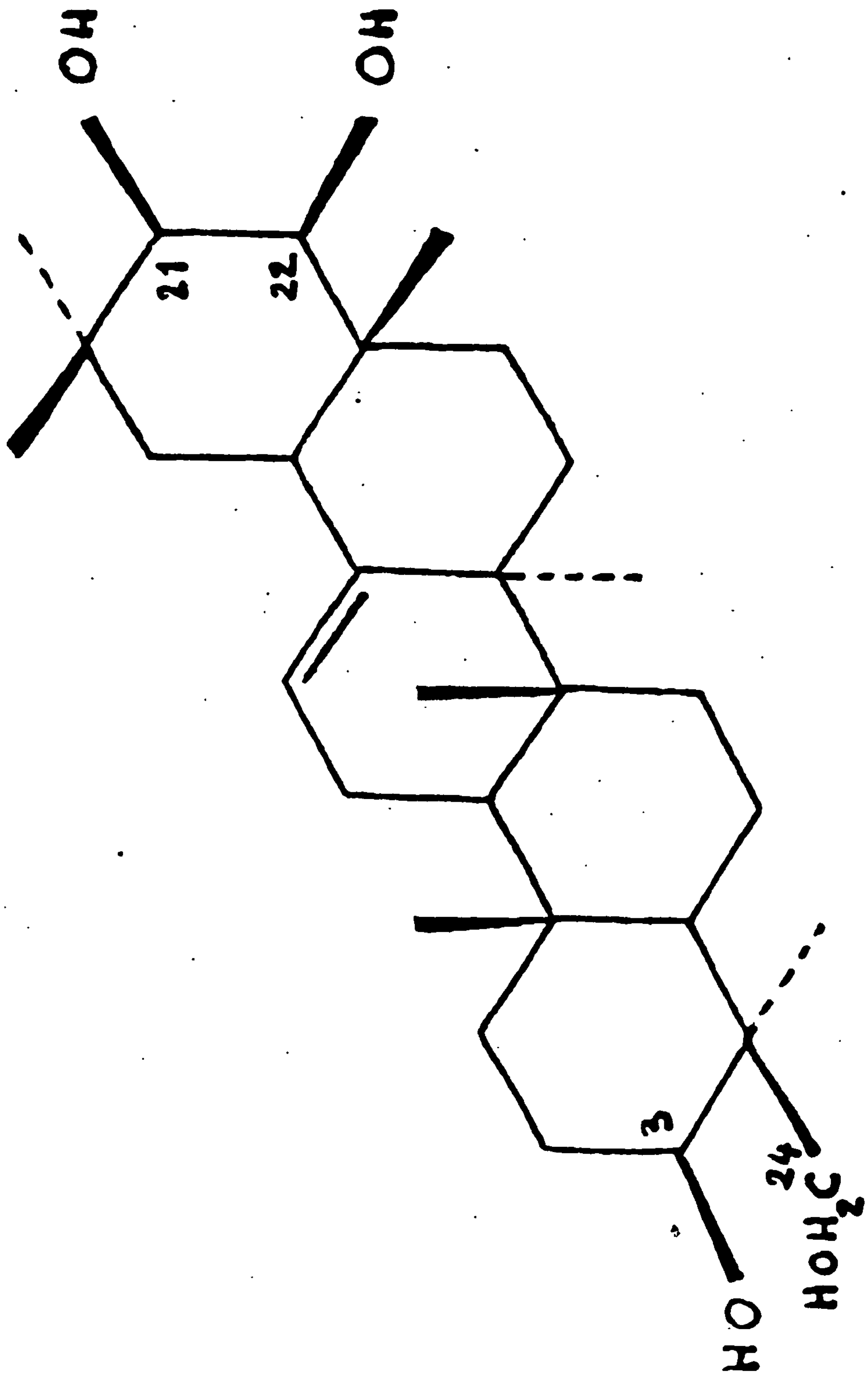

- soyasapogénol de type B

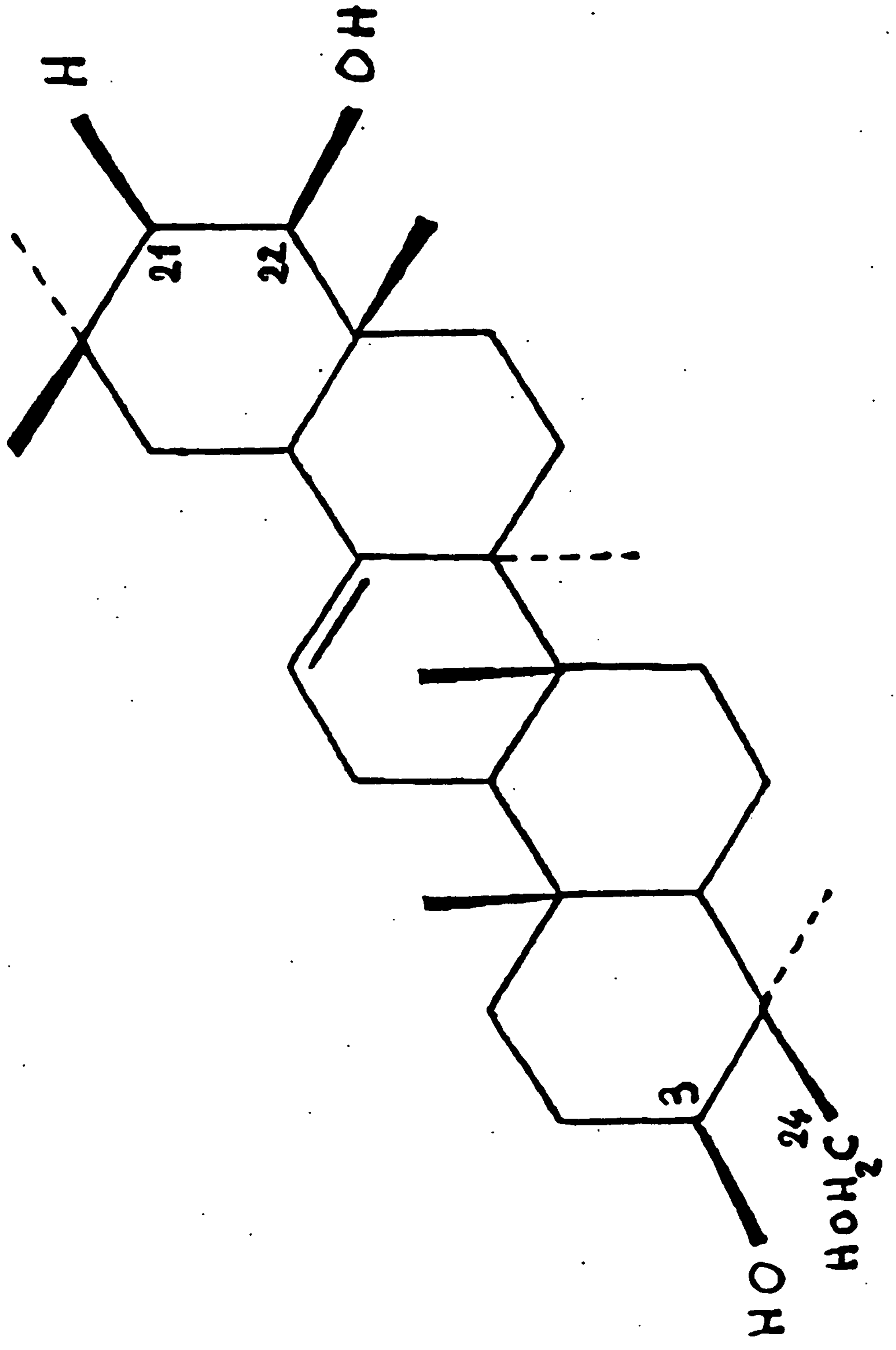

Les soyasapogénols ne comportent pas de substituant sucré et sont ainsi généralement dénommés aglycones. Par contre, ils peuvent être substitués par des sucres ou des chaînes de sucres et sont alors dénommés soyasaponines, qui peuvent ainsi être de type A ou de type B.

On citera par exemple :

| soyasapogénol A. aglycone (selon l'appendix 2 de Hostettman et Marston (1995) - Cambridge University Press) | | | |
|---|---|---|---|
| Nom | Structure | Extrait de plante | Référence bibliographique |
| N°517<br>Soyasaponine $A_2$ | 3-GlcA$^2$-Gal<br>22-Ara$^3$-Glc | Glycine max<br>(Leguminosae) | Kitagawa et al.<br>1985c |
| N°518<br>Soyasaponine $A_1$ | 3-GlcA$^2$-Gal$^2$-Glc<br>22-Ara$^3$-Glc | G. max<br>(Leguminosae) | Kitagawa et al.<br>1985a |
| N°519<br>Soyasaponine $A_3$ | 3-GlcA$^2$-Gal$^2$-Rha | G. max<br>(Leguminosae) | Curl et al. 1988b |
| soyasapogénol B. aglycone (selon l'appendix 2 de Hostettman et Marston (1995) - Cambridge University Press) | | | |
| Nom | Structure | Extrait de plante | Référence bibliographique |
| N°446 | 24-Glc | Phaseolus vulgaris<br>(Leguminosae) | Jain et al. 1988 |
| N°447<br>Soyasaponine IV | 3-GlcA$^2$-Ara | Glycine max<br>(Leguminosae) | Burrows et al.<br>1987 |
| N°448<br>Soyasaponine III | 3-GlcA$^2$-Gal | G. max<br>(Leguminosae) | Kitagawa et al.<br>1982 |
| N°449<br>Azukisaponine II | 3-GlcA$^2$-Glc | Vigna angularis<br>(Leguminosae)<br>Medicago sativa<br>(Luguminosae) | Kitagawa et al.<br>1983c<br>Kitagawa et al.<br>1988d |
| N°450<br>Phaseoluside A | 3-Glc$^4$-Glc<br>3<br>Glc | Phaseolus vulgaris<br>(Leguminosae) | Jain et al. 1991 |
| N°451<br>Soyasaponine II | 3-GlcA$^2$-Ara$^2$-Rha | Glycine max<br>(Leguminosae) | Kitagawa et al.<br>1982 |
| N°452 | 3-GlcA$^2$-Ara$^2$-Rha | Oxytropis<br>ochrocephala<br>(Leguminosae)<br>O.glabra<br>(Leguminasoae) | Sun et al. 1987<br><br><br>Sun et al. 1991 |
| N°453<br>Soyasaponine V | 3-GlcA$^2$-Gal$^2$-Glc | Glycine max<br>(Leguminosae)<br>Phaseolus vulgaris<br>(Leguminosae) | Taniyama et al.<br>1988b<br>Curl et al. 1988a |
| soyasapogénol B. aglycone (selon l'appendix 2 de Hostettman et Marston (1995) - Cambridge University Press) (suite) | | | |
| N°454<br>Soyasaponine I | 3-GlcA$^2$-Gal$^2$-Rha | Glycine max<br>(Leguminosae)<br>Medicago sativa<br>(Leguminosae)<br>Pisum sativum<br>(Leguminosae) | Kitagawa et al.<br>1976b<br>Kitagawa et al.<br>1988d<br>Price and Fenwick,<br>1984 |
| N°456<br>Azukisaponine V | 3-GlcA$^2$-Glc$^2$-Rha | Vigna angularis<br>(Leguminosae)<br>Medicago sativa | Kitagawa et al.<br>1983d<br>Kitagawa et al. |

(suite)

| soyasapogénol B. aglycone (selon l'appendix 2 de Hostettman et Marston (1995) - Cambridge University Press) (suite) | | | |
|---|---|---|---|
| | | (Leguminosae) | 1988d |
| N°457 | 3-GlcA$^4$-Glc$^2$-Rha | Oxytropis ochrocephala (Leguminosae) | Sun et al. 1987 |
| N°458 Astragaloside VIII | 3-GlcA$^2$-Xyl$^2$-Rha | Astragalus membranaceus (Leguminosae) | Kitagawa et al. 1983e |
| N°459 | 3-GlcA$^4$-Glc$^2$-Rha<br>2<br>Glc | Oxytropis glabra (Leguminosae) | Sun and Jia, 1990 |
| N°460 | 3-GlcA$^2$-Gal$^2$-Rha<br>6<br>Glc | Crotalaria albida (Leguminosae) | Ding et al. 1991a |

Les soyasaponines ou soyasapogénols précités triterpéniques peuvent être extraits à partir de toute partie de la plante mais seront de préférence extraits à partir des racines dans le cas de Medicago et des graines dans le cas du soja.

Les conditions d'extraction sont bien connues de l'homme du l'art qui pourra, par exemple, se reporter au document WO 92/09262 incorporé ici par référence.

Dans le cadre de l'utilisation conforme à la présente invention, la proportion en soyasaponine(s) ou en soyasapogénol (s) ou en extrait végétal en contenant est avantageusement comprise entre 0,001% et 5 %, mieux entre 0,01 et 2%, en poids, par rapport au poids total de la composition cosmétique ou pharmaceutique finale.

Il a également été découvert, de façon tout à fait inattendue que l'effet bénéfique des saponines et des sapogénols, quelle que soit leur nature, c'est-à-dire même si ladite saponine ou ledit sapogénol n'est pas de nature triterpénique, bien que l'emploi d'une saponine ou d'un sapogénol de nature triterpénique, de préférence de type A ou de type B, soit préféré, peut être sensiblement augmenté lorsque ces composés sont utilisés en association avec un ecdystéroïde ou un dérivé acétylé d'ecdystéroïde comme, en particulier, la β-ecdysone ou ecdystérone.

Les ecdystéroïdes, et en particulier l'ecdystérone sont des substances naturelles bien connues, présentes notamment chez les insectes et dans certaines plantes telles que Polypodium vulgare et Cyanotis arachnoidea.

Dans le cadre d'une utilisation associant une saponine ou un sapogénol et un ecdystéroïde, ces composés seront utilisés de préférence dans un rapport pondéral compris entre 1:10 et 10:1 et de préférence encore égal à 1:1.

Les soyasaponines ou soyasapogénols peuvent en outre être utilisées en association avec une autre substance active par exemple choisie parmi le groupe constitué par l'acide madécassique, l'acide asiatique, le madécassoside, l'asiaticoside, l'α-1-protéinase inhibiteur, les inhibiteurs de collagénase tels que l'acide rétinoïque, les inhibiteurs d'élastase, la lysine, la proline, le 2-oxoglutarate, le ginsénoside $R_0$, la vitamine D et ses dérivés, les vitamines, en particulier la vitamine A, B ou E ; les xanthines, la tyrosine ou ses dérivés comme par exemple le tyrosinate de glucose, la malyltyrosine, la quinine ou ses dérivés, les rubéfiants tels que le nicotinate de méthyle, des hydrolysats de kératine, des oligoéléments tels que le zinc, le sélénium, cuivre, des inhibiteurs de 5-α-réductase tels qu progestérone, l'acétate de cyprotérone, le minoxidil, l'acide azélaïque et ses dérivés, le 1,4-méthyl-4-azastéroïde, en particulier la 17-p-N,N-diéthylcarbamoyl-4-méthyl-4-aza-5-α-androstan-3-one, ou encore un extrait de Serenoa repens.

Dans ce cas, la concentration en cette (ou ces) autre(s) substance(s) active(s) est comprise entre 0,0001 % et 5 % en poids par rapport au poids de la composition finale.

Ces substances, incluant les soyasaponines ou soyasapogénols, peuvent éventuellement être incorporées au moins en partie dans des phases lamellaires lipidiques hydratées ou dans des vésicules de type liposomes.

Selon un deuxième aspect, la présente demande vise à couvrir de nouvelles compositions cosmétiques favorisant l'augmentation de la quantité de collagène IV dans la jonction dermo-épidermique.

Ces compositions sont essentiellement caractérisées par le fait qu'elles contiennent une quantité efficace de l'association d'au moins une soyasaponine ou un soyasapogénol, triterpénique, de type A ou de type B, avec au moins un ecdystéroïde ou au moins un dérivé acétylé d'ecdystéroïde, en particulier la β-ecdysone ou ecdystérone.

Dans ce cas, la soyasaponine ou le soyasapogénol précité et l'ecdystéroïde précité sont associés dans un rapport pondéral compris entre 1 : 10 et 10 : 1, et de préférence égal à 1 : 1.

Selon un troisième aspect, la présente demande vise à couvrir un procédé de traitement cosmétique non thérapeutique dans lequel on procède à l'administration topique à une personne en ayant besoin, d'une quantité efficace

d'au moins une soyasaponine ou d'au moins un soyasapogénol tel que défini précédemment en association avec un ecdystéroïde, en particulier l'ecdystérone, éventuellement en association avec une ou plusieurs autres substances actives telles que mentionnées ci-dessus.

D'autres buts, caractéristiques et avantages de l'invention apparaîtront clairement à la lecture de la description explicative qui va suivre faite en référence à plusieurs exemples donnés à titre d'illustration et qui ne sauraient donc en aucune façon limiter la portée de l'invention.

Dans les exemples, les pourcentages sont exprimés en poids, la température est la température ambiante, et la pression est la pression atmosphérique, sauf indication contraire. Dans le cas des extraits, les quantités de ceux-ci sont exprimées en poids sec.

Exemple 1

Mise en évidence de l'augmentation de la quantité de collagène IV avec des saponines extraites de graines de soja

Dans cet exemple, on utilise un extrait de graines de soja, disponible dans le commerce sous la référence SOY SELECT LOT 26040/2 auprès de la société italienne Indena.

Cet extrait contient selon les informations commerciales des saponines de la catégorie des "soyasaponines" en proportion de 29 à 30 % en poids sec, comprenant majoritairement des soyasapogénols du groupe B.

Afin de valider la pertinence du protocole expérimental choisi, une série de ces essais ont été réalisés avec un témoin positif, le Transforming Growth Factor - beta (TGF-beta), connu comme augmentant la quantité de collagène IV dans une culture de kératinocytes humains. On pourra se reporter à l'article de Köning A., Bruckner-Tuderman L. ; J. Cell. Biol. ; 1992 May ;117 (3) ;679-85.

1. Protocole du test

a. Provenance des kératinocytes :

Les cultures de kératinocytes humains normaux (KHN) sont établies à partir d'un prélèvement chirurgical de peau saine. Dans la présente étude, les tests ont été réalisés sur une souche cellulaire provenant d'un lifting d'une femme caucasienne de 44 ans, souche notée KH 44.

b. Conditions de culture :

Les kératinocytes sont cultivés dans le milieu de croissance K-SFM, qui est un milieu spécifique de culture des kératinocytes, SFM (Serum Free Medium) complet, complémenté en EGF (Epidermal Growth Factor) et en extrait pituitaire, commercialisé par la société Gibco, France. Les cellules ont été sous-cultivées une fois à partir de la primoculture selon un passage, noté P1.

c. Conditions de traitement :

L'ensemencement des cellules est réalisé en plaques de culture à 96 puits à raison de 25 000 KHN par puits dans le milieu K-SFM. Après 24 h d'incubation nécessaire à la bonne adhérence des cellules, le milieu d'ensemencement est retiré et remplacé par du milieu K-SFM dilué au 50$^{e}$ dans le même milieu de base, mais dépourvu de EGF et d'extrait pituitaire.

On ajoute alors le produit à tester selon l'invention que l'on dissout dans le DMSO à diverses concentrations reportées au tableau 1 ci-après, et que l'on cultive pendant 72 h.

Les témoins recevront la même quantité de solvant DMSO que pour le produit testé à 0,1 % final.

Les sumageants de culture sont récupérés pour réaliser un dosage ELISA comme expliqué ci-après.

d. Dosage ELISA du collagène IV :

Le protocole du dosage du collagène IV par une méthode ELISA est adapté de celui décrit par DUMAS M. et al. dans Mechanisms of Ageing and Development, 1994, 73, pages 179-187.

On utilise un anticorps anti-collagène IV humain fourni par l'Institut Pasteur, Lyon, France. Une courbe d'étalonnage est effectuée avec du collagène IV de placenta humain de chez Sigma. Parallèlement, un dosage global des protéines est effectué par la technique à l'acide bicinchoninique, kit BCA, commercialisé par la société Sigma, France, dans le but de rapporter la quantité de collagène IV au taux de protéines cellulaires.

Six cultures sont réalisées pour chacune des trois concentrations et pour l'essai témoin.

e. Expression des résultats et interprétation statistique

Les résultats de l'augmentation de la quantité de collagène IV dans une culture de kératinocytes sont exprimés en nanogrammes de collagène IV pour 100 µg de protéines après culture pendant 72h.
L'activité A du produit est exprimée en pourcentage et correspond à la formule suivante :

$$A = \left( \frac{Qs - Qt}{Qt} \right) \times 100$$

où :

- Qs est la quantité de collagène IV des KHN traités exprimée en ng pour 100 µg de protéines, après 72 heures.
- Qt est la quantité de collagène IV des KHN témoins DMSO exprimée en ng pour 100 µg de protéines, après 72 heures.

Les résultats obtenus sur les cultures traitées (n=6) et témoins (n=6) sont comparés par le test t de Student non apparié, le seuil de significativité étant p = 0,05.

2. Résultats-conclusion

Les résultats sont reportés au tableau 1 ci-après, à partir de la moyenne des mesures sur les différentes cultures :

TABLEAU 1

| PRODUIT | Q (ng de coll IV/100 µg de protéines ; à 72 h) | A (%) | S ignificativité p = 0,05 |
|---|---|---|---|
| témoin DMSO | 3,743±0,443 | 0 | |
| témoin positif (TGF-beta) 10 ng/ml | 5,615±0,595 | +50 | S |
| saponines de soja 2,5 µg/ml | 3,931±0,426 | +5 | NS |
| saponines de soja 5 µg/ml | 4,474±0,511 | +19 | S |
| saponines de soja 10 µg/ml | 4,762±0,591 | +27 | S |
| saponines de soja 25 µg/ml | 5,152±0,596 | +37,5 | S |
| S= significatif<br>NS = non significatif | | | |

On notera que le taux de collagène IV est exprimé en nanogrammes pour 100 µg de protéines après 72 h de contact avec les cellules.
Il apparaît clairement que les saponines testées augmentent significativement la quantité de collagène IV dans une culture de kératinocytes humains.
On notera également que le témoin positif (TGF-beta) répond significativement au protocole expérimental choisi, ce qui confirme sa validation et la pertinence des essais réalisés.
Ainsi, les saponines, en particulier celles extraites du soja, peuvent être utilisées en cosmétique ou en pharmacie ou dermopharmacie pour augmenter la quantité de collagène IV. Dans ce cas, elles permettent de renforcer la structure et les propriétés de la jonction dermo-épidermique, zone d'échange entre le derme et l'épiderme constituant des zones très importantes pour les processus de différentiation des kératinocytes.

Exemple 2

Mise en évidence de l'augmentation de la quantité de collagène IV en présence de saponines de Medicago

Cet essai est réalisé de la façon suivante :
Le produit à tester constitué par des saponines de racines de Medicago sativa, est disponible dans le commerce et commercialisé par la société Berkem, sous la forme d'une poudre.
Les tests ont été réalisés en aveugle, le produit étant dénommé saponine Medicago.

1. Protocole du test

a. Provenance des kératinocytes

Les cultures de kératinocytes humains normaux (KHN) sont établies à partir d'un prélèvement chirurgical de peau saine. Dans la présente étude, les tests ont été réalisés sur une souche cellulaire provenant d'un lifting d'une femme caucasienne de 48 ans, souche notée KHN 555.

b. Conditions de culture

Les kératinocytes sont cultivés de la même manière que celle décrite à l'exemple précédent, si ce n'est que les cellules ont été sous-cultivées 4 fois et récoltées au stade de la 4$^{e}$ sous-culture ou 4$^{e}$ passage, noté P4.

c. Conditions de traitement

L'ensemencement des cellules est réalisé en plaques de culture à 96 puits en raison de 30 000 KHN par puits dans le milieu K-SFM.

Après 24 h d'incubation nécessaire à la bonne adhérence des cellules, le milieu est remplacé par un milieu K-SFM dilué à 2 %, limitant la prolifération des kératinocytes.

Les produits selon l'invention sont préparés extemporanément dans le DMSO à une proportion respectivement de 2,5-10 et 25 mg/ml à partir du produit disponible dans le commerce, et ces solutions mères sont introduites dans le milieu d'étude à 0,1 % V/V final, soit des concentrations testées à 2,5-10 et 25 μg/ml. Le témoin reçoit l'excipient des produits, soit 0,1 % V/V de DMSO.

L'absence de cytotoxicité du produit selon l'invention aux différentes concentrations testées a été démontrée par ailleurs à l'aide des tests de viabilité XTT de chez BOEHRINGER, France.

Après 48 h de traitement, les surnageants en incubation sont prélevés en vue du dosage du collagène IV présent. Un dosage des protéines est effectué sur le tapis cellulaire restant dans les puits selon la méthode BCA de chez SIGMA, dans le but de ramener la quantité de collagène IV présent au taux de protéines cellulaires.

d. Dosage ELISA du collagène IV

Le protocole de dosage est le même que celui de l'exemple 1, mais les modifications suivantes ont été apportées dans la mise au point du dosage.

- Premier anticorps : anticorps monoclonal de lapin anti-collagène IV humain de l'INSTITUT PASTEUR DE LYON, Réf. 20411.
- Gamme étalon réalisée avec du collagène de type IV humain d'origine placentaire humaine, SIGMA référencé C5533.

e. Expression des résultats et interprétation statistique

Les résultats des expériences exprimés selon la méthode décrite au e) de l'exemple 1, font l'objet du tableau II ci-après.

2. Résultats-conclusion

Les résultats sont donnés dans le tableau II ci-après à partir de la moyenne des mesures sur les différentes cultures :

TABLEAU II

| PRODUITS | Q (ng de coll IV/ 100 μg prot. à 48 h) | A(%) | Significativité p = 0,05 |
|---|---|---|---|
| TEMOIN K-SFM 2 % + DMSO 0,1 % | 1,565+/-0,314 | 0 | |
| saponines de luzerne (2,5 μg/ml) | 1,745+/-0,409 | +11 | NS |
| saponines de luzerne (10 μg/ml) | 2,858+/-0,529 | +83 | S |

(suite)

| PRODUITS | Q (ng de coll IV/ 100 μg prot. à 48 h) | A(%) | Significativité p = 0,05 |
|---|---|---|---|
| saponines de luzerne (25 μg/ml) | 2,336+/-0,523 | +49 | S |
| S = significatif<br>NS : non significatif | | | |

On constate une augmentation significative de la quantité de collagène IV présent dans les cultures de kératinocytes humains normaux, réalisées en présence de saponines de racines de Medicago sativa ou luzerne. Les concentrations actives de saponines sont de 10 et 25 μg/ml avec le maximum d'activité à 10 μg/ml.

Exemple 3

Mise en évidence de l'augmentation de la quantité de collagène IV en présence de saponines extraites de graines de soja et de béta-ecdysone.

Le protocole est le même que celui décrit dans l'exemple 1, ceci près différence que l'extrait de graines de soja (référence SOY SELECT LOT 26040/2 disponible auprès de la société italienne Indena) est utilisé associé avec de la béta-ecdysone, dans un rapport pondéral 1/1 pour ces deux éléments, et que le temps de contact entre les produits à tester et les cellules est de 48 heures (au lieu de 72 heures).

Les résultats sont donnés dans le tableau III ci-après à partir de la moyenne des mesures sur les différentes cultures :

TABLEAU III

| PRODUIT | Q (ng de coll IV/100 μg de protéines ; à 48 h) | A (%) | Significativité p = 0,05 |
|---|---|---|---|
| témoin DMSO | 1,565±0,314 | 0 | |
| saponines de soja 2,5 μg/ml | 1,643±0.320 | +5 | NS |
| saponines de soja 2,5 μg/ml et béta-ecdysone 2,5 μg/ml | 2,441±0,360 | +56 | S |
| saponines de soja 5 μg/ml | 1,682±0,317 | +7.5 | NS |
| saponines de soja 5 μg/ml et béta-ecdysone 5 μg/ml | 2,081±0,340 | +33 | S |
| saponines de soja 10 μg/ml | 1,972±0,331 | +26 | S |
| saponines de soja 10 μg/ml et béta-ecdysone 10 μg/ml | 2,003±0,336 | +28 | S |
| saponines de soja 20 μg/ml | 1,975±0,335 | +26 | S |
| saponines de soja 20 μg/ml et béta-ecdysone 20 μg/ml | 1,998±0,342 | +27,5 | S |
| S= significatif .<br>NS = non significatif | | | |

On constate à la lecture du tableau III, que l'utilisation de l'association, ou combinaison, des saponines de soja avec de la béta-ecdysone, à certaines concentrations (2,5 et 5 μg/ml pour chacun des deux éléments) est encore plus efficace que l'utilisation des saponines de soja seules pour augmenter la quantité de collagène IV dans un milieu où sont présents des kératinocytes humains normaux.

La même expérience a été conduite en utilisant de la béta-ecdysone seule. Aucune augmentation de la quantité de collagène IV n'a été constatée.

Les inventeurs ont donc conclu qu'il existe un effet de synergie entre les saponines de soja et la béta-ecdysone pour ce qui est de la capacité de ces éléments à provoquer une augmentation de la quantité de collagène IV dans un milieu où sont présents des kératinocytes humains normaux.

La combinaison d'une saponine de soja avec la béta-ecdysone semble donc être très efficace comme moyen pour augmenter la quantité de collagène IV dans un milieu où sont présents des kératinocytes humains normaux.
Ainsi, l'association de saponine et d'ecdystéroïde selon l'invention présente un grand intérêt pour des applications cosmétiques.

Exemple 4 (exemple de référence non conforme à l'invention)

Crème luttant contre les marques du vieillissement

| | |
|---|---|
| Rétinol | 4000 UI |
| Saponines de soja de type B | 0,01 g |
| Glycérine | 3 g |
| Extrait de Commiphora mukkul | 0,1 g |
| Excipient émulsion fluide | qsp. 100 g |

A utiliser le soir pour traiter les rides, appliquer cette émulsion fluide en la faisant pénétrer.

Exemple 5 (exemple de référence non conforme à l'invention)

Crème anti-rides et préventive du photovieillissement

| | |
|---|---|
| Rétinol | 2000 UI |
| Palmitate de rétinol | 0,01 g |
| Palmitate d'ascorbyl | 0,1 g |
| Protéines de Blé | 3 g |
| Saponines de soja | 0,05 g |
| Madécassoside | 0,05 g |
| Parsol MCX | 7 g |
| Benzophénone 3 | 1 g |
| Beurre de Karité | 1 g |
| Excipient crème parfumée | qsp. 100 g |

Appliquer cette crème dès la fin d'après-midi, permet de réparer les rides de l'intérieur, et de protéger la peau des derniers rayons de soleil.

**Revendications**

1. Utilisation non thérapeutique d'au moins une soyasaponine ou un soyasapogénol de nature triterpénique de type A ou de type B, cosmétiquement acceptable, en particulier une soyasaponine ou un soyasapogénol triterpénique de type, A ou de type B extrait de plante, de préférence de soja ou d'une plante de type Medicago, ou d'un extrait végétal riche en de tels composés, en association avec au moins un ecdystéroïde ou avec au moins un dérivé acétylé d'ecdystéroïde, en particulier avec la beta-ecdysone ou ecdystérone, comme agent cosmétique destiné à augmenter la quantité de collagène IV dans la jonction dermo-épidermique, pour maintenir ou restaurer l'état physiologique de la jonction dermo-épidermique.

2. Utilisation selon la revendication 1, d'au moins une soyasaponine ou un soyasapogénol de nature triterpénique de type A ou B, extrait(e) d'une plante riche en de tels composés choisie parmi : Glycine max (soja), Phaseolus vulgaris, Phaseolus aureus, Phaseolus lunatus, Vicia faba, Lens culinaris, Cicer arietum, Vigna angularis, Vigna mungo, Oxytropis ochrocephala, Oxytropis glabra, Pisum sativum, Sophora flavescens Asparalus membranaceus, Crotalaria albida, Arachis hypogea, Galega officinalis, Wistaria brachybotrys, Trifolium repens, ou une plante du type Medicago,

en particulier Medicago alfalfa et Medicago sativa ou "luzerne".

3. Utilisation selon l'une quelconque des revendications précédentes d'au moins une soyasaponine ou un soyasapogénol triterpénique de type A ou B, extrait(e) d'une plante de type Medicago telle que Medicago alfalfa et Medicago sativa.

4. Utilisation selon l'une quelconque des revendications 1 ou 2 d'au moins une soyasaponine ou un soyasapogénol de type A ou B extrait(e) de soja.

5. Utilisation selon l'une quelconque des revendications précédentes, **caractérisée en ce que** la soyasaponine ou un soyasapogénol de type A ou B précité est extrait à partir de toute partie de la plante, de préférence à partir des racines dans le cas de Medicago et des graines dans le cas du soja.

6. Utilisation selon l'une quelconque des revendications précédentes, **caractérisée en ce que** la proportion en soyasaponine(s) ou en soyasapogénol (s) ou en extrait végétal en contenant est comprise entre 0,001 % et 5 %, mieux entre 0,01 et 2 %, en poids, par rapport au poids total de la composition cosmétique finale.

7. Utilisation selon l'une des revendications précédentes, **caractérisée en ce que** la saponine ou le sapogénol est utilisé en association avec au moins une autre substance active par exemple choisie parmi le groupe constitué par l'acide madécassique, l'acide asiatique, le madécassoside, l'asiaticoside, l'$\alpha$-1-protéinase inhibiteur, les inhibiteurs de collagénase tels que l'acide rétinoïque, les inhibiteurs d'élastase, la lysine, la proline, le 2-oxoglutarate, le ginsénoside $R_0$, la vitamine D et ses dérivés, les vitamines, en particulier la vitamine A, B ou E ; les xanthines, la tyrosine ou ses dérivés comme par exemple le tyrosinate de glucose, la malyltyrosine, la quinine ou ses dérivés, les rubéfiants tels que le nicotinate de méthyle, des hydrolysats de kératine, des oligoéléments tels que le zinc, le sélénium, cuivre, des inhibiteurs de 5-$\alpha$-réductase tels que progestérone, l'acétate de cyprotérone, le minoxidil, l'acide azélaïque et ses dérivés, le 1,4-méthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

8. Utilisation selon la revendication 7, **caractérisée en ce que** la concentration en cette (ou ces) autre(s) substance (s) active(s) est comprise entre 0,0001 % et 5 % en poids par rapport au poids de la composition finale.

9. Composition cosmétique favorisant l'augmentation de la quantité de collagène IV dans la jonction dermo-épidermique, **caractérisée en ce qu'**elle contient une quantité efficace de l'association d'au moins une soyasaponine ou un soyasapogénol tels que définis à l'une quelconque des revendications 1 à 6, avec au moins un ecdystéroïde, ou avec au moins un dérivé acétylé d'ecdystéroïde, en particulier avec la beta-ecdysone ou ecdystérone.

10. Composition selon la revendication 9, **caractérisée en ce que** la soyasaponine ou un soyasapogénol précité et l'ecdystéroïde précité sont associés dans un rapport pondéral compris entre 1 : 10 et 10 : 1, et de préférence égal à 1 : 1.

11. Procédé de traitement cosmétique non thérapeutique, **caractérisé en ce que** l'on procède à l'administration topique à une personne en ayant besoin, d'une quantité efficace d'au moins une soyasaponine ou d'au moins un soyasapogénol, tels que définis à l'une quelconque des revendications 1 à 6, en association avec au moins un ecdystéroïde ou avec au moins un dérivé acétylé d'ecdystéroïde, en particulier avec la beta-ecdysone ou ecdystérone , permettant d'augmenter la quantité de collagène IV dans la jonction dermo-épidermique, pour maintenir ou restaurer l'état physiologique de la jonction dermo-épidermique, ladite soyasaponine ou ledit soyasapogénol étant éventuellement administré en association avec une ou plusieurs autre(s) substance(s) active(s) telle(s) que définie(s) à la revendication 7.

12. Procédé selon la revendication 11, **caractérisé en ce que** le traitement cosmétique est un traitement anti-rides.

## Claims

1. Non-therapeutical use of at least one cosmetically acceptable soya saponin or soya sapogenol of triterpenic character of type A or B, particularly a soya saponin or soya sapogenol triterpenic of type A or B extracted from a plant, preferably from soya or a plant of the Medicago type, or of a plant extract rich in such compounds, in association with at least one ecdysteroid or with at least one acetylated derivative of ecdystéroide, in particular with beta-ecdysone or ecdystérone, as a cosmetic agent for increasing the amount of collagen IV in the dermo-epidermal junction to maintain or restore the physiological state of the dermo-epidermal junction.

2. Use according to claim 1 of at least one soya saponin or soya sapogenol of triterpenic character of type A or B extracted from a plant rich in such compounds and selected from Glycine max (soya), Phaseolus vulgaris, Phaseolus aureus, Phaseolus lunatus, Vicia faba, Lens culinaris, Cicer arietum, Vigna angularis, Vigna mungo, Oxytropis ochrocephala, Oxytropis glabra, Pisum sativum, Sophora flavescens, Asparalus membranaceus, Crotalaria albida, Arachis hypogea, Galega officinalis, Wistaria brachybotrys and Trifolium repens, or a plant of the Medicago type, particularly Medicago alfalfa and Medicago sativa, or "alfalfa".

3. Use according to any one of the preceding claims of at least one triterpenic soya saponin or soya sapogenol of type A or B extracted from a plant of the Medicago type, such as Medicago alfalfa and Medicago sativa.

4. Use according to any one of the claims 1 or 2 of at least one soya saponin or soya sapogenol of type A or B extracted from soya.

5. Use according to any one of the preceding claims, **characterized in that** the above-mentioned soya saponin or soya sapogenol of type A or B is extracted from any part of the plant, preferably from the roots in the case of Medicago and from the seeds in the case of soya.

6. Use according to any one of the preceding claims, **characterized in that** the proportion of soya saponin(s) or soya sapogenol(s), or of plant extract containing them, is ranging between 0.001% and 5% by weight, preferably between 0.01 and 2% by weight, based on the total weight of the final cosmetic composition.

7. Use according to one of the preceding claims, **characterized in that** the saponin or sapogenol is used in association with at least one other active substance selected e.g. from the group consisting of madecassic acid, asiatic acid, madecassoside, asiaticoside, α-1-proteinase inhibitor, collagenase inhibitors such as retinoic acid, elastase inhibitors, lysine, proline, 2-oxoglutarate, ginsenoside $R_{0,}$ vitamin D and derivatives thereof, other vitamins, particularly vitamin A, B or E, xanthines, tyrosine or derivatives thereof, for example glucose tyrosinate or malyltyrosine, quinine or derivatives thereof, rubefacients such as methyl nicotinate, keratin hydrolyzates, trace elements such as zinc, selenium or copper, 5-α-reductase inhibitors such as progesterone or cyproterone acetate, minoxidil, azelaic acid and derivatives thereof, 1,4-methyl-4-azasteroid, particularly 17β-N,N-diethylcarbamoyl-4-methyl-4-aza-5-α-androstan-3-one, or an extract of Serenoa repens.

8. Use according to claim 13, **characterized in that** the concentration of this (these) other active substance(s) is ranging between 0.0001% and 5% by weight, based on the weight of the final composition.

9. Cosmetic composition which promotes an increase in the amount of collagen IV in the dermo-epidermal junction, **characterized in that** it contains an effective amount of at least one soya saponin or soya sapogenol, as defined in any one of claims 1 to6, in association with at least one ecdysteroid or at least one acetylated ecdysteroid derivative, particularly with beta-ecdysone or ecdysterone.

10. Composition according to claim9, **characterized in that** the said saponin or sapogenol and the said ecdysteroid are associated in a weight ratio ranging between 1:10 and 10:1 and preferably of 1:1.

11. Method of cosmetic non-therapeutic treatment, **characterized in that** an effective amount of at least one soya saponin or at least one soya sapogenol, as defined in any one of claims 1 to 6, is administered topically to a person in need thereof, in association with at least one ecdysteroid or at least one acetylated ecdysteroid derivative, particularly with beta-ecdysone or ecdysterone for increasing the amount of collagen IV in the dermo-epidermal junction to maintain or restore the physiological state of the dermo-epidermal junction, said soya saponin or soya sapogenol being eventually administered in association with one or several other active substance(s) such as defined in claim 7.

12. Method according to claim 11, wherein the cosmetic treatment is an anti-wrinkle treatment.

**Patentansprüche**

1. Nicht therapeutische Verwendung wenigstens eines kosmetisch verträglichen triterpenartigen Sojasaponins oder

Sojasapogenols Typ A oder Typ B, insbesondere eines Triterpensojasaponins oder -sojasapogenols Typ A oder Typ B, das aus einer Pflanze, vorzugsweise aus Soja oder aus einer Pflanze vom Typ Medicago extrahiert ist, oder eines Pflanzenextraktes, der reich an solchen Verbindungen ist, in Kombination mit wenigstens einem Ecdysteroid oder mit wenigstens einem acetylierten Ecdysteroid-Derivat, insbesondere mit Beta-Ecdyson oder Ecdysteron, als kosmetischer Wirkstoff, der dazu bestimmt ist, die Menge an Kollagen IV in der dermoepidermalen Junktionszone zu erhöhen, um den physiologischen Zustand der dermoepidermalen Junktionszone aufrechtzuerhalten oder wiederherzustellen.

2. Verwendung nach Anspruch 1, wenigstens eines triterpenartigen Sojasaponins oder Sojasapogenols Typ A oder B, das aus einer Pflanze, die reich an solchen Verbindungen ist, extrahiert ist, welche ausgewählt ist aus: Glycine max (Soja), Phaseolus vulgaris, Phaseolus aureus, Phaseolus lunatus, Vicia faba, Lens culinaris, Cicer arietum, Vigna angularis, Vigna mungo, Oxytropis ochrocephala, Oxytropis glabra, Pisum sativum, Sophora flavescens, Asparalus membranaceus, Crotalaria albida, Arachis hypogea, Galega officinalis, Wistaria brachybotrys, Trifolium repens, oder einer Pflanze vom Typ Medicago, insbesondere Medicago alfalfa und Medicago sativa oder "Luzerne".

3. Verwendung nach einem der vorhergehenden Ansprüche, wenigstens eines Triterpensojasaponins oder -sojasapogenols Typ A oder B, das aus einer Pflanze vom Typ Medicago, wie Medicago alfalfa und Medicago sativa extrahiert ist.

4. Verwendung nach einem der Ansprüche 1 oder 2, wenigstens eines Sojasaponins oder Sojasapogenols Typ A oder B, das aus Soja extrahiert ist.

5. Verwendung nach einem der vorhergehenden Ansprüche, **dadurch gekennzeichnet, daß** das Sojasaponin oder ein vorgenannte Sojasapogenol Typ A oder B aus jedwedem Teil der Pflanze, vorzugsweise aus den Wurzeln im Fall von Medicago und aus den Samen im Fall von Soja extrahiert ist.

6. Verwendung nach einem der vorhergehenden Ansprüche, **dadurch gekennzeichnet, daß** der Anteil an Sojasaponin(en) oder an Sojasapogenol(en) oder an diese enthaltenden Pflanzenextrakt im Bereich zwischen 0,001 und 5 Gew.-%, besser zwischen 0,01 und 2 Gew.-%, bezogen auf das Gesamtgewicht der kosmetischen Endzusammensetzung liegt.

7. Verwendung nach einem der vorhergehenden Ansprüche, **dadurch gekennzeichnet, daß** das Saponin oder Sapogenol in Kombination mit wenigstens einem weiteren Wirkstoff verwendet wird, der beispielsweise ausgewählt ist aus der Gruppe bestehend aus Madekassische Säure, Asiatische Säure, Madecassosid, Asiaticosid, $\alpha$-1-Proteinase-Inhibitor, Kollagenase-Inhibitoren wie Retinolsäure, Elastase-Inhibitoren, Lysin, Prolin, 2-Oxoglutarat, Ginsenosid $R_0$, Vitamin D und seinen Derivaten, Vitaminen, insbesondere Vitamin A, B oder E, Xanthinen, Thyrosin oder seinen Derivaten, wie zum Beispiel Glucosetyrosinat, Malyltyrosin, Chinin oder seinen Derivaten, den Rubefazienzien, wie Methylnicotinat, Keratinhydrolysaten, Oligoelementen, wie Zink, Selen, Kupfer, 5-$\alpha$-Reduktase-Inhibitoren, wie Progesteron, Cyproteronacetat, Minoxidil, Azelainsäure und ihren Derivaten, 1,4-Methyl-4-azasteroid, insbesondere 17-$\beta$-N,N-Diethylcarbamoyl-4-methyl-4-aza-5-$\alpha$-androstan-3-one, oder aber einem Extrakt aus Serenoa repens.

8. Verwendung nach Anspruch 7, **dadurch gekennzeichnet, daß** die Konzentration an diesem (oder diesen) weiteren Wirkstoff(en) im Bereich zwischen 0,0001 und 5 Gew.-% bezogen auf das Gewicht der Endzusammensetzung liegt.

9. Kosmetische Zusammensetzung, die die Steigerung der Menge an Kollagen IV in der dermoepidermalen Junktionszone begünstigt, **dadurch gekennzeichnet, daß** sie eine wirkungsvolle Menge der Kombination aus wenigstens einem Sojasaponin oder einem Sojasapogenol, wie sie in einem der Ansprüche 1 bis 6 definiert sind, mit wenigstens einem Ecdysteroid oder mit wenigstens einem acetylierten Ecdysteroid-Derivat, insbesondere mit Beta-Ecdyson oder Ecdysteron enthält.

10. Zusammensetzung nach Anspruch 9, **dadurch gekennzeichnet, daß** das Sojasaponin oder ein vorgenanntes Sojasapogenol und das vorgenannte Ecdysteroid in einem Gewichtsverhältnis zwischen 1 : 10 und 10 : 1 und vorzugsweise gleich 1 : 1 kombiniert werden.

11. Verfahren zur nicht therapeutischen kosmetischen Behandlung, **dadurch gekennzeichnet, daß** einer Person, die dies benötigt, eine wirkungsvolle Menge wenigstens eines Sojasaponins oder wenigstens eines Sojasapogenols, wie sie in einem der Ansprüche 1 bis 6 definiert sind, in Kombination mit wenigstens einem Ecdysteroid oder mit wenigstens einem acetylierten Ecdysteroid-Derivat, insbesondere mit Beta-Ecdyson oder Ecdysteron topisch verabreicht wird, die ermöglicht, die Menge an Kollagen IV in der dermoepidermalen Junktionszone zu erhöhen, um den physiologischen Zustand der dermoepidermalen Junktionszone aufrechtzuerhalten oder wiederherzustellen, wobei das Sojasaponin oder das Sojasapogenol eventuell in Kombination mit einem oder mehreren weiteren Wirkstoff(en), wie er (sie) in Anspruch 7 definiert ist (sind), verabreicht wird.

**12.** Verfahren nach Anspruch 11, **dadurch gekennzeichnet, daß** die kosmetische Behandlung eine Antifalten-Behandlung ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9625143 A
- WO 9728814 A
- EP 0826367 A
- WO 9209262 A
- US 4254067 A, Arichi
- WO 9404132 A
- WO 9003778 A
- WO 9962481 A


### Littérature non-brevet citée dans la description


- **GOMES DA SILVA ; CORTESI.** *La Rivista Italiana delle Sostanze Grasse,* 1997, vol. 74 (7), 311-314
- **A.Y. LEUNG.** Encyclopedia of Common Natural Ingredients used in foods, drugs and cosmetics. WILEY, Interscience, 1996, 284-286
- **GEORGES MASSIOT.** *J. Agric.Food Chem.,* 1988, vol. 36, 902-909
- **GEORGES MASSIOT ; CATHERINE LAVAUD ; DOMINIQUE GUILLAUME ; LOUISETTE LE MEN-OLIVIER.** *J. Agric. Food Chem.,* 1988, vol. 36, 902-909
- **HOSTETTMAN K. ; MARSTON A.** Saponins. Cambridge University Press, 1995
- **KÖNING A. ; BRUCKNER-TUDERMAN L.** *J. Cell. Biol.,* Mai 1992, vol. 117 (3), 679-85
- **DUMAS M. et al.** *Mechanisms of Ageing and Development,* 1994, 179-187